# EUROPÄISCHE PATENTSCHRIFT

(11) **EP 3 135 259 B1**
(45) Veröffentlichungstag und Bekanntmachung des Hinweises auf die Patenterteilung: **09.05.2018**
(21) Anmeldenummer: 15182182.4
(22) Anmeldetag: 24.08.2015
(51) Int. Cl.: A61F 5/37

(54) **VORRICHTUNG ZUM FIXIEREN EINES PATIENTEN AUF EINER UNTERLAGE**
DEVICE FOR FIXING A PATIENT TO A SUPPORT
DISPOSITIF DESTINE A ATTACHER UN PATIENT SUR UN SUPPORT

(43) Veröffentlichungstag der Anmeldung: 01.03.2017
(73) Patentinhaber: Pöltenstein, Markus, 1130 Wien (AT)
(72) Erfinder: Pöltenstein, Markus, 1130 Wien (AT)
(74) Vertreter: KLIMENT & HENHAPEL

(56) Entgegenhaltungen:
- EP-A1- 1 523 966
- US-A- 5 148 815
- US-A1- 2013 019 882
- US-B1- 6 202 647
- US-B1- 6 823 870
- US-B2- 8 092 406

## Beschreibung

### GEBIET DER ERFINDUNG

Die vorliegende Erfindung betrifft eine Vorrichtung zum Fixieren eines Patienten auf einer Unterlage, insbesondere auf einem Bett.

### STAND DER TECHNIK

Bei der Behandlung von Patienten kann es in verschiedenen Situationen vorkommen, dass der Patient auf einer Unterlage fixiert werden muss, beispielsweise um das Herunterfallen von der Unterlage auszuschließen oder um Patienten mit starkem Bewegungsdrang vor unkontrollierten Bewegungen, insbesondere unmittelbar nach einer erfolgten Operation, zu schützen. Derlei Situationen können z.B. in der Intensivmedizin, Anästhesie, Notaufnahme, Ambulanz, Psychiatrie oder in Aufwachräumen eintreten.

Um Patienten mit deren Oberkörper an einem Bett zu fixieren, sind Brustgurte bekannt, die zusätzlich mit Schultergurten kombiniert werden können. Die Kombination der Gurte bzw. die Befestigung am Bett erfolgt z.B. mittels Schlösser, die in dafür vorgesehenen Löchern der Gurte angeordnet werden können.

Die bekannten Lösungen weisen eine Reihe von Nachteilen auf. Die Anordnung von Gurten über der Brust schränkt zum einen das Freiheitsgefühl des Patienten ein. Zum anderen erschwert oder verhindert diese Anordnung den Zugang zum Brust- und Bauchbereich des Patienten, z.B. bei notwendigen Behandlungen in diesen Bereichen.

Weiters müssen die bekannten Befestigungs- bzw. Verbindungsmöglichkeiten mittels Schlösser als umständlich angesehen werden, was einerseits die Sicherheit der Anwendung gefährden kann und andererseits in manchen Fällen zu unerwünschtem Zeitverlust führen kann.

Da die Gurte in der Lage sein müssen, mitunter hohe Zugkräfte aufzunehmen, sind die Gurte entsprechend mechanisch stabil ausgeführt, sodass die Gurte von den Patienten als unbequem empfunden werden.

Beispielsweise schlägt die US 6 202 647 B1 einen einen Fixierungsgurt zum Fixieren eines Patienten auf einer Unterlage vor, welcher einen Hauptgurt mit Kontaktabschnitten und zwei endseitig angeordneten Anbindungsabschnitten aufweist. Im angelegten Zustand verläuft der Gurt unter den Achseln durch, über die Schulter und hinter dem Kopf.

### AUFGABE DER ERFINDUNG

Es ist daher Aufgabe der vorliegenden Erfindung eine Vorrichtung zum Fixieren eines Patienten auf einer Unterlage zur Verfügung zu stellen, die die oben genannten Nachteile vermeidet. Insbesondere soll die Vorrichtung für den Patienten so bequem wie möglich sein, diesem ein größtmögliches Freiheitsgefühl erlauben und die Zugänglichkeit zum Bauch- und Brustbereich ermöglichen. Weiters soll die Vorrichtung eine einfache, schnelle und sichere Immobilisierung des Patienten erlauben.

### DARSTELLUNG DER ERFINDUNG

Die Erfindung betrifft eine Vorrichtung zum Fixieren eines Patienten auf einer Unterlage, insbesondere auf einem Bett, die Vorrichtung umfassend einen Hauptgurt mit einem eine Polsterung aufweisenden Kontaktabschnitt und einem ersten Anbindungsabschnitt in einem ersten Endbereich des Hauptgurts und einem zweiten Anbindungsabschnitt in einem zweiten Endbereich des Hauptgurts, wobei der Kontaktabschnitt entlang des Verlaufs des Hauptgurts gesehen zwischen dem ersten Endbereich und dem zweiten Endbereich angeordnet ist, wobei der Kontaktabschnitt so dimensioniert ist, dass in einem Anwendungszustand der Vorrichtung der Kontaktabschnitt derart am Patienten anordenbar ist, dass der Kontaktabschnitt am Rücken des Patienten, weiters unter den Achseln kranial und schließlich dorsal hinter dem Kopf des Patienten, sich in einem Kreuzpunkt kreuzend, oder dorsal hinter dem Kopf des Patienten, sich in einem Näherungspunkt annähernd, verläuft. "Kranial" bedeutet hierbei zum Schädel bzw. Kopf hin, "dorsal" bedeutet zum Rücken hin.

Der Kontaktabschnitt dient also zur Kontaktierung des Patienten. Die Polsterung garantiert im Anwendungszustand eine größtmögliche Bequemlichkeit für den Patienten und stellt sicher, dass der Hauptgurt bzw. der Kontaktabschnitt im Anwendungszustand den Patienten nicht einschneiden kann.

Bei der durch die Dimensionierung bzw. Auslegung des Kontaktabschnitts ermöglichten Anordnung des Hauptgurts bzw. Kontaktabschnitts am Patienten bleibt im Anwendungszustand der Brust- und Bauchbereich des Patienten frei. Eine Behandlung in diesen Bereichen kann daher problemlos auch bei fixiertem Patienten vorgenommen werden. Zudem gewährleisten der freie Brust- und Bauchbereich ein maximales Freiheitsgefühl für den Patienten.

Für das Anlegen des Hauptgurts bzw. des Kontaktabschnitts sind grundsätzlich zwei Varianten denkbar: einerseits kann der Kontaktabschnitt, nachdem er unter den Achseln durch und über die Schultern verläuft, sich hinter dem Kopf in einem Kreuzpunkt kreuzend verlaufen, wobei sich der Kreuzpunkt alleine durch das Überkreuzen des Hauptgurts und das Festziehen der Endbereiche einstellen lässt. Andererseits kann der Kontaktabschnitt dorsal hinter dem Kopf des Patienten, sich in einem Näherungspunkt annähernd, vorzugsweise berührend, verlaufen. Der Näherungspunkt ist dabei jener Punkt an dem die beiden von der jeweiligen Schulter des Patienten kommenden Teile des Kontaktabschnitts den geringsten Abstand voneinander haben, wobei es auch denkbar ist, dass die beiden Teile einander im Näherungspunkt berühren. Nach dem Erreichen des Näherungspunkts entfernen sich die beiden Teile des Kontaktabschnitts in der Regel wieder voneinander, um die Spannkraft zu erhöhen. Um die Annäherung im Näherungspunkt zu erreichen, ist erfindungsgemäß ein Fixierelement vorgesehen durch welches die beiden Teile des Kontaktabschnitts geführt sind, wodurch der Abstand zwischen den beiden Teilen des Kontaktabschnitts einstellbar ist.

Unabhängig davon, ob der Kontaktabschnitt nun sich in einem Kreuzpunkt kreuzend oder sich in einem Näherungspunkt annähernd verläuft, bildet sich im Anwendungszustand durch die Lage des Punktes hinter dem Kopf des Patienten ein dreiecksförmiger (bei Kreuzung oder Berührung) oder ein trapezförmiger Verlauf (bei bloßer Annäherung) des Kontaktabschnitts zwischen den Schultern des Patienten und dem Kopf des Patienten aus, der ein Herauswinden der Arme des Patienten und damit ein Befreien aus der Fixierung verhindert.

Nachdem der Hauptgurt bzw. der Kontaktabschnitt in der beschriebenen Weise dem Patienten angelegt worden ist, wird der Hauptgurt durch Ziehen an den Endbereichen festgezogen und an der Unterlage befestigt. Zur Befestigung sind am Hauptgurt die Anbindungsabschnitte vorgesehen, welche der Anbindung des Hauptgurts an Befestigungselemente dienen, wobei die Befestigungselemente wiederum an der Unterlage befestigt sind.

Die durch die Dimensionierung bzw. Auslegung des Kontaktabschnitts ermöglichte Anordnung des Hauptgurts bzw. Kontaktabschnitts am Patienten bewirkt, dass die Endbereiche bzw. die Anbindungsabschnitte des Hauptgurts vom Kopf des Patienten in kranialer Richtung beabstandet sind. Auf diese Weise wird der Möglichkeit vorgebeugt, dass im Anwendungszustand der Patient die Vorrichtung bzw. die Anbindungsabschnitte von den Befestigungselementen selbst lösen kann. Somit kann im Anwendungszustand eine sichere Fixierung des Oberkörpers des Patienten auf der Unterlage garantiert werden.

Um im Anwendungszustand den Kreuzpunkt oder den Näherungspunkt hinter dem Kopf des Patienten zu fixieren, ist erfindungsgemäß ein Fixierelement vorgesehen. Auf diese Weise kann sichergestellt werden, dass die Lage des Kreuzpunkts oder des Näherungspunkts sich im Anwendungszustand nicht verschiebt, insbesondere dass sich die Lage des Kreuzpunkts oder des Näherungspunkts dauerhaft hinter dem Kopf des Patienten befindet. Zum einen kann hierdurch sichergestellt werden, dass der Kreuzpunkt oder der Näherungspunkt so nahe zum Nacken des Patienten angeordnet ist, dass es für den Patienten unmöglich ist, seinen Kopf zwischen dem Kreuzpunkt oder dem Näherungspunkt bzw. dem Hauptgurt und der Unterlage zu positionieren. Insbesondere kann hierdurch eine Strangulation des Patienten ausgeschlossen werden. Zum anderen kann die Lage des Kreuzpunkts oder des Näherungspunkts zum Nacken des Patienten so gewählt werden, dass kein unangenehmer Druck gegen den Nacken oder Kopf des Patienten entsteht, wenn der Patient mit seinem Kopf auf der Unterlage liegt bzw. der Kopf durch die Unterlage gestützt wird. In der Praxis kann ein Kopfkissen zwischen dem Hauptgurt im Bereich des Kreuzpunkts oder des Näherungspunkts und dem Kopf des Patienten angeordnet werden, sodass der Kopf des Patienten direkt das Kopfkissen berührt bzw. auf diesem liegt, was für den Patienten besonders bequem ist. Für die Fixierung des Kreuzpunkts oder des Näherungspunkts bei einander berührenden Teilen des Kontaktabschnitts hat sich ein wiederverschließbares Fixierelement als besonders vorteilhaft herausgestellt. Sind die beiden von den Schultern des Patienten kommenden Teile des Kontaktabschnitts jedoch voneinander beabstandet, so kann beispielsweise ein Fixierelement zum Einsatz kommen, welches zwei voneinander mittels eines Mittelteils beabstandete Ösen zur Aufnahme jeweils eines Teils des Kontaktabschnitts umfasst.

Das Fixierelement wird vorzugsweise nach dem Festziehen und Befestigen des Hauptgurts angebracht. Die Wiederverschließbarkeit des Fixierelements erlaubt ein beliebiges Lösen und Wiederverschließen des Fixierelements, beispielsweise um die Lage des Kreuzpunkts bei Bedarf nachzujustieren.

Bei einer bevorzugten Ausführungsform der erfindungsgemäßen Vorrichtung ist es vorgesehen, dass der Kontaktabschnitt so dimensioniert ist, dass im Anwendungszustand der Vorrichtung der Kontaktabschnitt derart am Patienten anordenbar ist, dass der Kontaktabschnitt am Rücken des Patienten unterhalb von dessen Schulterblättern verläuft. Dies bewirkt eine Anordnung des Kontaktabschnitts am Patienten, die ein ungewolltes Aufbäumen sowie ein Hinunterrutschen des Patienten verhindert. Zudem ist diese Anordnung besonders bequem für den Patienten. Die Lagestabilität des Patienten kann dabei durch zusätzliche Fixierungen an Händen und Füßen weiter verbessert werden.

Um ein einfach handzuhabendes Fixierelement zu realisieren, ist es bei einer bevorzugten Ausführungsform der erfindungsgemäßen Vorrichtung vorgesehen, dass das Fixierelement als flexibles Band ausgebildet ist.

Um eine besonders einfache Handhabung zu gestatten, ist es bei einer bevorzugten Ausführungsform der erfindungsgemäßen Vorrichtung vorgesehen, dass das Fixierelement einen Klettverschluss zum Wiederverschließen aufweist. Der Klettverschluss weist vorzugsweise ein Hakenband und ein Flauschband auf. Der Klettverschluss ermöglicht ein extrem rasches Schließen und auch Öffnen des Fixierelements. Zudem können über den Klettverschluss große Zugkräfte parallel zu jenen Flächen, auf denen der Klettverschluss bzw. das Flauschband und/oder das Hakenband angeordnet sind, übertragen werden, sodass ein sicheres Fixieren des Kreuzpunkts durch das Fixierelement gewährleistet ist.

Um ein bequemes Zuziehen des Fixierelements rund um den Kreuzpunkt zu ermöglichen, ist es bei einer bevorzugten Ausführungsform der erfindungsgemäßen Vorrichtung vorgesehen, dass das Fixierelement zumindest einen D-Ring, vorzugsweise aus Metall aufweist.

Im Falle eines bandförmigen Fixierelements mit Klettverschluss ist der D-Ring vorzugsweise an einem Ende des Fixierelements angeordnet. Nachdem das Fixierelement um den Kreuzpunkt herum angeordnet ist, kann das andere Ende durch den D-Ring geführt werden. Durch ein darauffolgendes Ziehen am anderen Ende wird das Fixierelement festgezogen. Durch eine darauffolgende Fixierung des anderen Endes mittels des Klettverschlusses wird das Fixierelement schließlich verschlossen und fixiert.

Denkbar wäre, insbesondere bei einer bandförmigen Ausführung des Fixierelements, auch eine Ausführung mit zwei D-Ringen. In diesem Fall könnte auf einen Klettverschluss auch verzichtet werden, da das Fixierelement mittels der beiden D-Ringe zuverlässig verschlossen werden könnte.

Die Ausführung des mindestens einen D-Rings aus Metall garantiert dabei ein leichtes Einfädeln des anderen Endes durch den mindestens einen D-Ring sowie eine hohe mechanische Stabilität des festgezogenen und verschlossenen Fixierelements.

Bei einer bevorzugten Ausführungsform der erfindungsgemäßen Vorrichtung ist es vorgesehen, dass das Band des Fixierelements aus Mikrofaser gefertigt ist. Durch die Verwendung von Mikrofaser kann eine besonders hohe Flexibilität des Bands bzw. des Fixierelements sichergestellt werden. Gleichzeitig kann durch diese Materialwahl gewährleistet werden, dass das Band im Wesentlichen undehnbar ist. Schließlich kann durch die Verwendung von Mikrofaser Latexfreiheit des Fixierelements garantiert werden.

Die Anbindungsabschnitte können grundsätzlich beliebige Mittel zur Verbindung mit den Befestigungselementen aufweisen. Um ein besonders schnelles und einfaches Verbinden der Anbindungsabschnitte mit den Befestigungselementen, aber auch ein schnelles und einfaches Lösen dieser Verbindung zu ermöglichen, ist es bei einer bevorzugten Ausführungsform der erfindungsgemäßen Vorrichtung vorgesehen, dass die Anbindungsabschnitte jeweils einen Klettverschluss aufweisen, um die Anbindung des Hauptgurts an Befestigungselemente zu ermöglichen. Entsprechend ist es bei einer besonders bevorzugten Ausführungsform der erfindungsgemäßen Vorrichtung vorgesehen, dass die Befestigungselemente jeweils einen Klettverschluss zur Anbindung des jeweiligen Anbindungsabschnitts des Hauptgurts aufweisen. Somit wird ein Zusammenwirken der Klettverschlüsse der Anbindungselemente mit den Klettverschlüssen der Befestigungselemente ermöglicht.

Der Klettverschluss weist vorzugsweise ein Hakenband und/oder ein Flauschband auf, wobei eine Art am Anbindungsabschnitt und die andere Art am Befestigungselement vorgesehen ist. Besonders bevorzugt sind an den Anbindungsabschnitten Flauschbänder vorgesehen, da es beim Anlegen des Hauptgurts am Patienten zu Kontakten zwischen Patient und den Anbindungsabschnitten kommen kann und sich Flauschbänder für den Patienten meist angenehmer anfühlen als Hakenbänder.

Der Klettverschluss ermöglicht ein extrem rasches Verbinden und auch Lösen der Anbindungsabschnitte und Befestigungselemente. Zudem können mittels des Klettverschlusses große Zugkräfte parallel zu jenen Flächen, auf denen der Klettverschluss bzw. das Flauschband und/oder das Hakenband angeordnet sind, übertragen werden. Entsprechend sicher kann der Patient fixiert werden bzw. kann ein Verrutschen des Patienten bei festgezogenem Hauptgurt vermieden werden. Gleichzeitig wird die Flexibilität des Hauptgurts durch die Klettverschlüsse nicht eingeschränkt, sodass das Anliegen des Gurts am Patienten vom Patienten in der Regel nicht als unangenehm empfunden wird.

Um besonders hohe Kräfte mittels der Klettverschlüsse übertragen zu können, müssen die Flächen, auf denen der jeweilige Klettverschluss bzw. das Flauschband und/oder das Hakenband angeordnet sind, entsprechend groß gestaltet werden. Zudem sollen die Anbindungsabschnitte ausreichend lang sein, um eine Größenverstellung zu ermöglichen. Gleichzeitig soll aber auch gewährleistet sein, dass ein direkter Kontakt zwischen dem Patient und den Anbindungsabschnitten vermieden wird, d.h. der Patient soll nur mit dem für ihn bequemen Kontaktabschnitt in Berührung kommen. Daher ist es bei einer bevorzugten Ausführungsform der erfindungsgemäßen Vorrichtung vorgesehen, dass der erste Anbindungsabschnitt und/oder der zweite Anbindungsabschnitt jeweils eine Länge aufweisen, die 1/10 bis 1/5 einer Gesamtlänge des Hauptgurts beträgt. Die Gesamtlänge des Hauptgurts kann dabei typischerweise von 220 cm bis 300 cm betragen, vorzugsweise von 240 cm bis 280 cm. Insbesondere bei besonders großgewachsenen Patienten sind auch größere Längen des Hauptgurts denkbar. Insbesondere bei besonders kleingewachsenen Patienten, wie z.B. bei Kindern, sind auch kleinere Längen des Hauptgurts denkbar. Je nach Anwendungsfall können die Anbindungsabschnitte vorzugsweise auch kürzer oder länger gestaltet werden.

Die Befestigungselemente können grundsätzlich mit beliebigen Mitteln an der Unterlage befestigt werden. Um ein besonders schnelles und einfaches Befestigen der Befestigungselemente mit der Unterlage sowie ein schnelles und einfaches Lösen dieser Verbindung zu ermöglichen, ist es bei einer bevorzugten Ausführungsform der erfindungsgemäßen Vorrichtung vorgesehen, dass die Befestigungselemente jeweils als Band ausgebildet sind und jeweils einen Klettverschluss zur Befestigung des jeweiligen Befestigungselements an der Unterlage aufweisen. Vorzugsweise weist dabei jedes Befestigungselement für die Befestigung an der Unterlage sowohl ein Hakenband als auch ein Flauschband auf.

Bei einer bevorzugten Ausführungsform der erfindungsgemäßen Vorrichtung ist es vorgesehen, dass die Befestigungselemente jeweils als Band ausgebildet sind, welches Band aus Mikrofaser gefertigt ist. Durch die Verwendung von Mikrofaser kann eine besonders hohe Flexibilität des Bands bzw. des jeweiligen Befestigungselements sichergestellt werden, was die Handhabbarkeit erhöht und ein einfaches und flexibles Befestigen des Befestigungselements an der Unterlage ermöglicht. Gleichzeitig kann durch diese Materialwahl gewährleistet werden, dass das Band im Wesentlichen undehnbar ist. Schließlich kann durch die Verwendung von Mikrofaser Latexfreiheit der Befestigungselemente garantiert werden.

Bei einer bevorzugten Ausführungsform der erfindungsgemäßen Vorrichtung ist es vorgesehen, dass der Hauptgurt zumindest abschnittsweise aus Mikrofaser gefertigt ist. Dadurch kann eine hohe Flexibilität des Hauptgurts erreicht werden. Gleichzeitig kann durch diese Materialwahl gewährleistet werden, dass der Hauptgurt zumindest abschnittsweise im Wesentlichen undehnbar ist.

Mikrofaser eignet sich insbesondere als Material zur Ausbildung des Kontaktabschnitts, da für den Patienten ein angenehmes Gefühl auf der Haut erzielt und gleichzeitig Latexfreiheit garantiert werden kann. Um die Polsterung zu ermöglichen, kann der Kontaktabschnitt im Wesentlichen als Mikrofaserband ausgebildet sein, welches mit einem Schlauch aus einem gepolsterten Material überzogen ist. Das Mikrofaserband ist vorzugsweise latexfrei und besonders bevorzugt im Wesentlichen undehnbar. Der Schlauch ist vorzugsweise latexfrei. Besonders bevorzugt ist der Schlauch aus Mikrofaser gefertigt.

Analog ist es bei einer besonders bevorzugten Ausführungsform der erfindungsgemäßen Vorrichtung vorgesehen, dass die Anbindungsabschnitte des Hauptgurts jeweils als Band ausgebildet sind, welches Band aus Mikrofaser gefertigt ist. Durch die Verwendung von Mikrofaser kann eine besonders hohe Flexibilität des Bands bzw. der Anbindungsabschnitte sichergestellt werden, was die Handhabbarkeit erhöht und ein einfaches Verbinden mit den Befestigungselementen ermöglicht. Gleichzeitig kann durch diese Materialwahl gewährleistet werden, dass das Band im Wesentlichen undehnbar ist. Schließlich kann durch die Verwendung von Mikrofaser Latexfreiheit der Anbindungsabschnitte garantiert werden.

Weiters ist zur Fixierung eines Patienten auf einer Unterlage erfindungsgemäß ein System vorgesehen, das System umfassend eine erfindungsgemäße Vorrichtung und als Unterlage ein Bett mit einer Matratze und einem Bettrost, auf welchem die Matratze im Anwendungszustand der Vorrichtung aufliegt, wobei im Anwendungszustand der Vorrichtung der Hauptgurt mit den Anbindungsabschnitten an Befestigungselemente angebunden ist, welche am Bettrost befestigt sind. Durch die Befestigung der Befestigungselemente am Bettrost wird sichergestellt, dass der Hauptgurt nicht ungewollt gespannt oder gelockert oder gar gelöst wird, wenn die Neigung des Bettrosts im Kopfbereich, also die Neigung eines Kopfteils des Betts, geändert wird.

### KURZE BESCHREIBUNG DER FIGUREN

Die Erfindung wird nun anhand eines Ausführungsbeispiels näher erläutert. Die Zeichnungen sind beispielhaft und sollen den Erfindungsgedanken zwar darlegen, ihn aber keinesfalls einengen oder gar abschließend wiedergeben.

Dabei zeigt:
- Fig. 1: eine schematische axonometrische Ansicht eines Hauptgurts einer erfindungsgemäßen Vorrichtung
- Fig. 2: eine schematische axonometrische Ansicht eines Fixierelements der erfindungsgemäßen Vorrichtung
- Fig. 3: eine schematische axonometrische Ansicht eines Befestigungselements der erfindungsgemäßen Vorrichtung
- Fig. 4: eine schematische Aufsicht auf einen Patienten, der auf einem Bett mittels der erfindungsgemäßen Vorrichtung in einem Anwendungszustand fixiert ist
- Fig. 5: eine Detailansicht aus Fig. 4, wobei jedoch der Patient sowie eine Matratze des Betts nicht dargestellt sind

### WEGE ZUR AUSFÜHRUNG DER ERFINDUNG

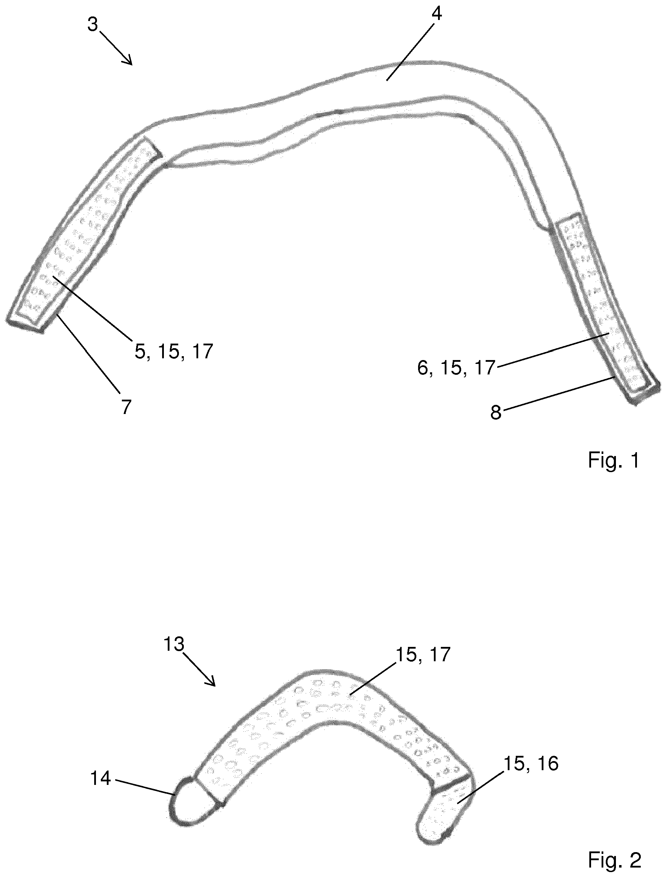

Fig. 1 zeigt einen Hauptgurt 3 einer erfindungsgemäßen Vorrichtung zum Fixieren eines Patienten 1 auf einer Unterlage, insbesondere auf einem Bett 2. Der Hauptgurt 3 weist einen Kontaktabschnitt 4 auf, der entlang des Verlaufs des Hauptgurts 3 gesehen zwischen einem ersten Endbereich 7 und einem zweiten Endbereich 8 angeordnet ist.

Der Kontaktabschnitt 4 dient zur Kontaktierung des Patienten 1 und ist derart dimensioniert bzw. ausgelegt, dass in einem Anwendungszustand der Vorrichtung der Kontaktabschnitt 4 derart am Patienten 1 anordenbar ist, dass der Kontaktabschnitt 4 am Rücken des Patienten 1, weiters unter den Achseln kranial und schließlich dorsal hinter dem Kopf des Patienten 1, sich in einem Kreuzpunkt 9 kreuzend verläuft. Vorzugsweise ist der Verlauf des Kontaktabschnitts 4 am Rücken des Patienten 1 dabei unterhalb der Schulterblätter.

In einer alternativen nicht dargestellten Ausführungsform ist der Kontaktabschnitt 4 derart am Patienten anordenbar, dass der Kontaktabschnitt 4 am Rücken des Patienten 1, weiters unter den Achseln kranial und schließlich dorsal hinter dem Kopf des Patienten 1, sich in einem Näherungspunkt annähernd oder berührend verlaufend angeordnet ist.

D.h. der Kontaktabschnitt 4 muss einerseits lang genug sein, um besagte Anordnung am Patienten 1 zu ermöglichen. Andererseits muss der Kontaktabschnitt 4 eine Breite und eine Höhe aufweisen, die derart gestaltet sind, dass ein problemloses Durchführen bzw. Verlaufen unter den Achseln des Patienten 1 ermöglicht wird und gleichzeitig die mechanische Stabilität, insbesondere Zugfestigkeit des Hauptgurts 3 groß genug ist, um eine sichere Fixierung des Patienten 1 zu gewährleisten.

Wie in Fig. 1 erkennbar ist, ist der Kontaktabschnitt 4 gepolstert ausgeführt, um ein Einschneiden des Patienten 1 zu unterbinden. Hierzu ist der Kontaktabschnitt 4 als Mikrofaserband ausgeführt, welches mit einem Schlauch aus einem gepolsterten Material überzogen ist. Das Mikrofaserband ist vorzugsweise latexfrei und besonders bevorzugt im Wesentlichen undehnbar. Der Schlauch ist vorzugsweise latexfrei. Besonders bevorzugt ist der Schlauch aus Mikrofaser gefertigt. Es sind aber selbstverständlich auch andere Ausführungsvarianten denkbar, z.B. kann es sich bei dem Kontaktabschnitt 4 alternativ auch nur um ein gepolstertes Band handeln.

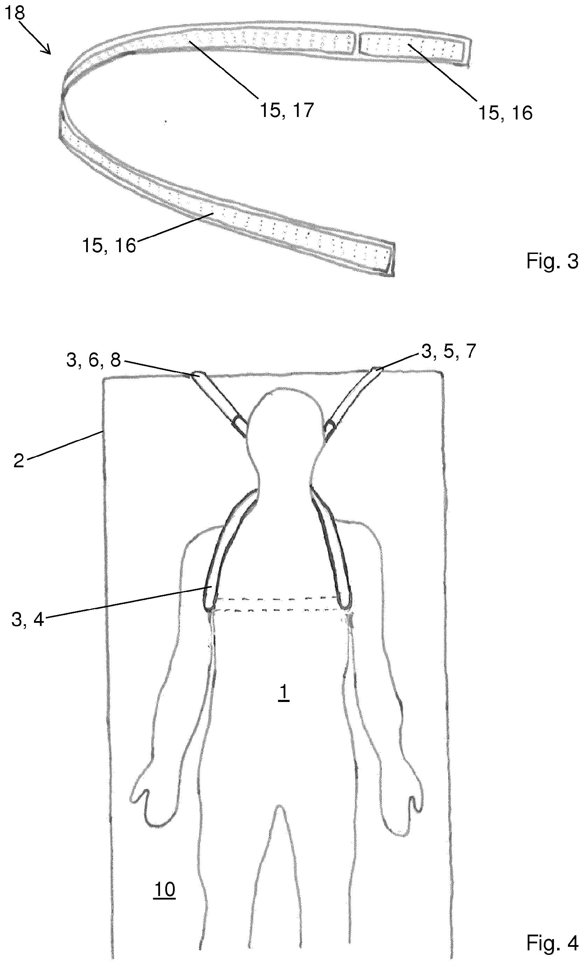

Im ersten Endbereich 7 ist ein erster Anbindungsabschnitt 5 zur Anbindung des Hauptgurts 3 an ein Befestigungselement 18 (vgl. Fig. 3) vorgesehen, im zweiten Endbereich 8 ein zweiter Anbindungsabschnitt 6 zur Anbindung des Hauptgurts 3 an ein weiteres Befestigungselement 18'. Die Anbindungsabschnitte 5, 6 bzw. die Endbereiche 7, 8 sind als ungepolsterte, flexible, latexfreie und im Wesentlichen undehnbare Bänder aus Mikrofaser ausgeführt, ebenso die Befestigungselemente 18, 18'. Die Befestigungselemente 18, 18' dienen zur Befestigung am Bett 2.

Die Anbindungsabschnitte 5, 6 weisen jeweils einen Klettverschluss 15 auf, der mit einem Klettverschluss 15 der Befestigungselemente 18, 18' zusammenwirken kann. Hierzu ist es beim gezeigten Ausführungsbeispiel vorgesehen, dass der Klettverschluss 15 der Anbindungsabschnitte 5, 6 jeweils als Flauschband 17 ausgeführt ist. Die Befestigungselemente 18, 18' weisen entsprechend jeweils ein Hakenband 16 als Gegenstück auf. In Fig. 3 ist erkennbar, dass dieses Hakenband 16 im Wesentlichen eine ganze Seite des Befestigungselements 18 ausfüllt, um ein großflächiges Verbinden mit dem Flauschband 17 des Anbindungsabschnitts 5 zu ermöglichen, wodurch wiederum besonders hohe Zugkräfte parallel zu den Flächen, auf denen die Hakenbänder 16 und Flauschbänder 17 angeordnet sind, mittels des Klettverschlusses 15 übertragen werden können. Dies gilt analog für das Befestigungselement 18'. Entsprechend groß sind auch die Anbindungsabschnitte 5, 6 dimensioniert und weisen vorzugsweise eine Länge auf, die 1/10 bis 1/5 der Gesamtlänge des Hauptgurts 3 beträgt.

Die Gesamtlänge des Hauptgurts 3 kann dabei typischerweise von 220 cm bis 300 cm betragen, vorzugsweise von 240 cm bis 280 cm. Die Befestigungselemente 18, 18' sind typischerweise jeweils 60 cm bis 90 cm lang.

Eine Breite des Hauptgurts 3 beträgt typischerweise zwischen 4 cm und 12 cm. Vorzugsweise sind dabei die Anbindungsabschnitte 5, 6 etwas breiter gestaltet sind als der Kontaktabschnitt 4, beispielsweise sind die Anbindungsabschnitte 5, 6 zwischen 6 cm und 12 cm breit und der Kontaktabschnitt zwischen 4 cm und 8 cm.

Eine Höhe des Kontaktabschnitts 4 beträgt typischerweise 0,5 cm bis 2,5 cm. Eine Höhe der Anbindungsabschnitte 5, 6 beträgt typischerweise 0,3 cm bis 0,7 cm.

Wie in Fig. 3 weiters ersichtlich ist, weist das Befestigungselement 18 auf der anderen Seite ebenfalls einen Klettverschluss 15 auf, und zwar sowohl ein Flauschband 17 als auch ein Hakenband 16. Dies gilt analog für das Befestigungselement 18'. Dieser Klettverschluss 15 ermöglicht eine einfache Befestigung der Befestigungselemente 18, 18' am Bett 2.

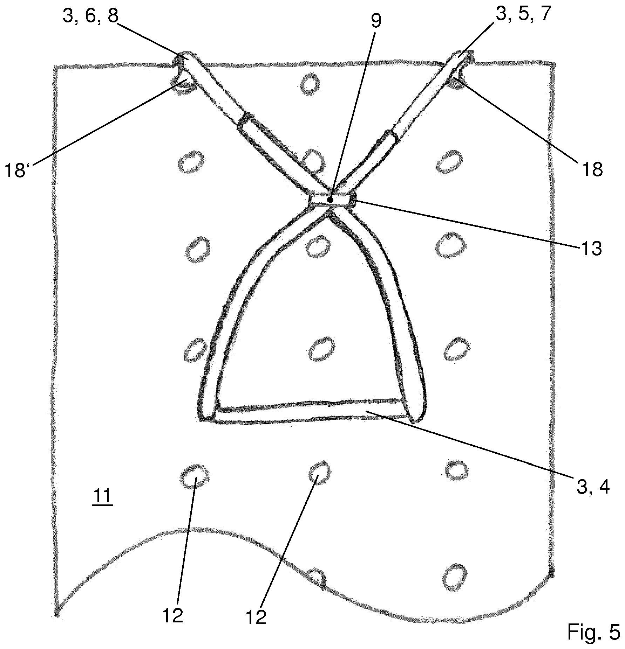

Dabei werden die Befestigungselemente 18, 18' vorzugsweise an einem Bettrost 11 des Betts 2 befestigt, auf welchem Bettrost 11 eine Matratze 10 des Betts 2 aufliegt. Durch die Befestigung der Befestigungselemente 18, 18' am Bettrost 11 wird sichergestellt, dass der Hauptgurt 3 nicht ungewollt gespannt oder gelockert oder gar gelöst wird, wenn die Neigung des Bettrosts 11 im Kopfbereich, also die Neigung eines Kopfteils des Betts 2, geändert wird. Fig. 5 illustriert diese Befestigung, wobei die Befestigungselemente 18, 18' jeweils durch ein Belüftungsloch 12 des Bettrosts 11 am oberen Ende des Bettrosts 11 geführt und mittels des Klettverschlusses am Bettrost 11 fixiert sind.

Nach der Befestigung der Befestigungselemente 18, 18' am Bettrost 11 und der Auflage der Matratze 10 auf den Bettrost 11 wird ein auf der Matratze 10 liegender Patient 1 fixiert, wie folgt: Zunächst wird der Oberkörper des Patienten 1 aufgerichtet. Der Hauptgurt 3 wird mit seinem Kontaktabschnitt 4 unterhalb der Schulterblätter am Rücken des Patienten 1 angelegt und rechts und links unter den Achseln kranial, d.h. zum Schädel bzw. Kopf hin, weitergeführt. Danach werden die Endbereiche 7, 8 dorsal, d.h. zum Rücken hin, weitergeführt und hinter dem Kopf des Patienten 1 gekreuzt, wodurch sich zwei Abschnitte des Kontaktabschnitts 4 im Kreuzpunkt 9 kreuzen. Der Hauptgurt 3 wird nun durch Ziehen an den Endbereichen 7, 8 festgezogen, und der erste Anbindungsabschnitt 5 wird an das Befestigungselement 18 angebunden und der zweite Anbindungsabschnitt 6 an das Befestigungselement 18'. Hierdurch wird der Oberkörper des Patienten 1 auf dem Bett 2 bzw. der Matratze 2 fixiert, wie in Fig. 4 illustriert ist, wobei der Verlauf des Hauptgurts 3 bzw. des Kontaktabschnitts 4 am Rücken des Patienten 1 durch strichlierte Linien angedeutet ist.

Um die Lage des Kreuzpunkts 9 zu fixieren, ist ein Fixierelement 13 vorgesehen, das in Fig. 2 illustriert ist. Im gezeigten Ausführungsbeispiel ist das Fixierelement 13 als ungepolstertes, flexibles, latexfreies und im Wesentlichen undehnbares Band aus Mikrofaser ausgeführt. Auf einer Seite weist das Fixierelement einen Klettverschluss 15 auf, wobei auf dieser Seite sowohl ein Flauschband 17 als auch ein Hakenband 16 vorgesehen sind. Weiters weist das Fixierelement 13 an einem Ende einen D-Ring 14 aus Metall auf.

Am anderen Ende des Fixierelements 13 ist kein D-Ring 14 vorgesehen. Das Hakenband 16 ist im Bereich des anderen Endes angeordnet. Das Flauschband 17 ist entlang des Verlaufs des Fixierelements 13 gesehen zwischen dem Hakenband 16 und dem D-Ring 14 angeordnet.

Zur Lagefixierung des Kreuzpunkts 9 mittels des Fixierelements 13 wird das Fixierelement 13 um den im Kreuzpunkt 9 gekreuzten Hauptgurt 3 gelegt. Daraufhin wird das andere Ende durch den D-Ring 14 geführt. Durch Zug am anderen Ende wird das Fixierelement 13 zusammengezogen, woraufhin das Fixierelement 13 mittels des Klettverschlusses 15 geschlossen wird. Während dieser Lagefixierung des Kreuzpunkts 9 wird der Kopf des Patienten 1 angehoben bzw. hebt der Patient 1 den Kopf. In Fig. 5 ist die Anordnung des Fixierelements 13 am Hauptgurt 3 bzw. die Fixierung der Lage des Kreuzpunkts 9 ersichtlich.

In der zuvor erwähnten alternativen Ausführungsvariante, in der der Kreuzpunkt durch den Näherungspunkt ersetzt ist, kann ebenfalls ein Fixierelement 13 der gleichen Bauart eingesetzt sein, wobei das Fixierelement 13 in diesem Fall die im Anwendungszustand durch die Verzurrung erzeugten nach außen wirkenden Kräfte aufnehmen muss. Insbesondere wenn es im Näherungspunkt zu keinem Kontakt der beiden Teile des Kontaktabschnitts 4 miteinander kommen soll, ist ein alternatives Fixierelement vorzusehen, welches etwa zwei über einen Mittelteil miteinander verbundene Ösen zur Aufnahme jeweils eines Teils des Kontaktabschnitts 4 umfasst.

### BEZUGSZEICHENLISTE

- 1: Patient
- 2: Bett
- 3: Hauptgurt
- 4: Kontaktabschnitt
- 5: Erster Anbindungsabschnitt
- 6: Zweiter Anbindungsabschnitt
- 7: Erster Endbereich des Hauptgurts
- 8: Zweiter Endbereich des Hauptgurts
- 9: Kreuzpunkt
- 10: Matratze
- 11: Bettrost
- 12: Belüftungsloch des Bettrosts
- 13: Fixierelement
- 14: D-Ring des Fixierelements
- 15: Klettverschluss
- 16: Hakenband des Klettverschlusses
- 17: Flauschband des Klettverschlusses
- 18, 18': Befestigungselement

## Patentansprüche

1. Vorrichtung zum Fixieren eines Patienten (1) auf einer Unterlage, insbesondere auf einem Bett (2), die Vorrichtung umfassend einen Hauptgurt (3) mit einem eine Polsterung aufweisenden Kontaktabschnitt (4) und einem ersten Anbindungsabschnitt (5) in einem ersten Endbereich (7) des Hauptgurts (3) und einem zweiten Anbindungsabschnitt (6) in einem zweiten Endbereich (8) des Hauptgurts (3), wobei der Kontaktabschnitt (4) entlang des Verlaufs des Hauptgurts (3) gesehen zwischen dem ersten Endbereich (7) und dem zweiten Endbereich (8) angeordnet ist, wobei die Länge, Breite und Höhe des Kontaktabschnitts (4) so dimensioniert sind, dass in einem Anwendungszustand der Vorrichtung der Kontaktabschnitt (4) derart am Patienten (1) anordenbar ist, dass der Kontaktabschnitt (4) am Rücken des Patienten (1), weiters unter den Achseln kranial und schließlich dorsal hinter dem Kopf des Patienten (1), sich in einem Kreuzpunkt (9) kreuzend, oder dorsal hinter dem Kopf des Patienten (1), sich in einem Näherungspunkt annähernd, verläuft,
**dadurch gekennzeichnet, dass**
ein Fixierelement (13) vorgesehen ist, um im Anwendungszustand den Kreuzpunkt (9) oder den Näherungspunkt zu fixieren.

2. Vorrichtung nach Anspruch 1, **dadurch gekennzeichnet, dass** die Länge, Breite und Höhe des Kontaktabschnitts (4) so dimensioniert sind, dass im Anwendungszustand der Vorrichtung der Kontaktabschnitt (4) derart am Patienten (1) anordenbar ist, dass der Kontaktabschnitt (4) am Rücken des Patienten (1) unterhalb von dessen Schulterblättern verläuft.

3. Vorrichtung nach einem der Ansprüche 1 bis 2, **dadurch gekennzeichnet, dass** das Fixierelement (13) wiederverschließbar ausgebildet ist.

4. Vorrichtung nach einem der Ansprüche 1 bis 3, **dadurch gekennzeichnet, dass** das Fixierelement (13) als flexibles Band ausgebildet ist.

5. Vorrichtung nach Anspruch 4, **dadurch gekennzeichnet, dass** das Fixierelement (13) einen Klettverschluss (15) zum Wiederverschließen aufweist.

6. Vorrichtung nach einem der Ansprüche 4 bis 5, **dadurch gekennzeichnet, dass** das Fixierelement (13) zumindest einen D-Ring (14), vorzugsweise aus Metall aufweist.

7. Vorrichtung nach einem der Ansprüche 4 bis 6, **dadurch gekennzeichnet, dass** das Band des Fixierelements (13) aus Mikrofaser gefertigt ist.

8. Vorrichtung nach einem der Ansprüche 1 bis 7, **dadurch gekennzeichnet, dass** die Anbindungsabschnitte (5, 6) jeweils einen Klettverschluss (15, 17) aufweisen, um die Anbindung des Hauptgurts (3) an Befestigungselemente (18, 18') zu ermöglichen.

9. Vorrichtung nach einem der Ansprüche 1 bis 8, **dadurch gekennzeichnet, dass** der Hauptgurt (3) zumindest abschnittsweise aus Mikrofaser gefertigt ist.

10. Vorrichtung nach Anspruch 9, **dadurch gekennzeichnet, dass** die Anbindungsabschnitte (5, 6) des Hauptgurts (3) jeweils als Band ausgebildet sind, welches Band aus Mikrofaser gefertigt ist.

11. Vorrichtung nach einem der Ansprüche 1 bis 10, **dadurch gekennzeichnet, dass** der erste Anbindungsabschnitt (5) und/oder der zweite Anbindungsabschnitt (6) jeweils eine Länge aufweisen, die 1/10 bis 1/5 einer Gesamtlänge des Hauptgurts (3) beträgt.

12. System umfassend eine Vorrichtung nach einem der Ansprüche 1 bis 11 und als Unterlage ein Bett (2) mit einer Matratze (10) und einem Bettrost (11), auf welchem die Matratze (10) im Anwendungszustand der Vorrichtung aufliegt, wobei im Anwendungszustand der Vorrichtung der Hauptgurt (3) mit den Anbindungsabschnitten (5, 6) an Befestigungselemente (18, 18') angebunden ist, welche am Bettrost (11) befestigt sind.

13. System nach Anspruch 12, **dadurch gekennzeichnet, dass** die Befestigungselemente (18, 18') jeweils einen Klettverschluss (15, 16) zur Anbindung des jeweiligen Anbindungsabschnitts (5, 6) des Hauptgurts (3) aufweisen.

14. System nach einem der Ansprüche 12 bis 13, **dadurch gekennzeichnet, dass** die Befestigungselemente (18, 18') jeweils als Band ausgebildet sind und jeweils einen Klettverschluss (15, 16, 17) zur Befestigung des jeweiligen Befestigungselements (18, 18') an der Unterlage (2) aufweisen.

15. System nach einem der Ansprüche 12 bis 14, **dadurch gekennzeichnet, dass** die Befestigungselemente (18, 18') jeweils als Band ausgebildet sind, welches Band aus Mikrofaser gefertigt ist.

## Claims

1. Device for fixing a patient (1) on a base, in particular on a bed (2), the device comprising a main belt (3) with a padded contact section (4) and a first attachment section (5) in a first end region (7) of the main belt (3) and a second attachment section (6) in a second end region (8) of the main belt (3), wherein the contact section (4), as seen along the course of the main belt (3), is arranged between the first end region (7) and the second end region (8), wherein the length, width and height of the contact section (4) are dimensioned such that in an application state of the device, the contact section (4) can be arranged on the patient (1) such that the contact section (4) extends on the back of the patient (1), further under the armpits cranially and finally dorsally behind the head of the patient (1), crossing in a crossing point (9), or dorsally behind the head of the patient(1), approximating in one approximation point, **characterized in that** a fixing element (13) is provided to fix the crossing point (9) or the approximation point in the application state.

2. Device according to claim 1, **characterized in that** the length, width and height of the contact section (4) are dimensioned such that in the application state of the device, the contact section (4) can be arranged on the patient (1) such that the contact section (4) on the back of the patient (1) runs below the shoulder blades.

3. Device according to one of the claims 1 to 2, **characterized in that** the fixing element (13) is formed to be reclosable.

4. Device according to one of the claims 1 to 3, **characterized in that** the fixing element (13) is designed as a flexible band.

5. Device according to claim 4, **characterized in that** the fixing element (13) has a hook-and-loop fastener (15) for reclosing.

6. Device according to one of the claims 4 to 5, **characterized in that** the fixing element (13) has at least one D-ring (14), preferably made of metal.

7. Device according to one of the claims 4 to 6, **characterized in that** the band of the fixing element (13) is made of microfiber.

8. Device according to one of the claims 1 to 7, **characterized in that** the attachment sections (5, 6) each have a hook-and-loop fastener (15, 17) to allow the attachment of the main belt (3) to fastening elements (18, 18').

9. Device according to one of the claims 1 to 8, **characterized in that** the main belt (3) is at least partially made of microfiber.

10. Device according to claim 9, **characterized in that** the attachment sections (5, 6) of the main belt (3) are each formed as a band, which band is made of microfiber.

11. Device according to one of the claims 1 to 10, **characterized in that** the first attachment section (5) and/or the second attachment section (6) each have a length which is 1/10 to 1/5 of an overall length of the main belt (3) .

12. System comprising a device according to one of the claims 1 to 11 and as a support a bed (2) with a mattress (10) and a bed frame (11) on which the mattress (10) rests in the application state of the device, wherein in the application state of the device the main belt (3) is attached with the attachment sections (5, 6) to fastening elements (18, 18') which are fastened to the bed frame (11).

13. System according to claim 12, **characterized in that** the fastening elements (18, 18') each have a hook-and-loop fastener (15, 16) for attaching the respective attachment section (5, 6) of the main belt (3).

14. System according to one of the claims 12 to 13, **characterized in that** the fastening elements (18, 18') are each formed as a band and in each case comprise a hook-and-loop fastener (15, 16, 17) for fastening the respective fastening element (18, 18') to the base (2).

15. System according to one of the claims 12 to 14, **characterized in that** the fastening elements (18, 18') are each formed as a band, which band is made of microfiber.

## Revendications

1. Dispositif pour la fixation d'un patient (1) sur un support, en particulier sur un lit (2), lequel dispositif comprend une sangle principale (3) avec une partie de contact (4) rembourrée et une première partie d'attache (5) dans une première zone d'extrémité (7) de la sangle principale (3) et une deuxième partie d'attache (6) dans une deuxième zone d'extrémité (8) de la sangle principale (3), dans lequel la partie de contact (4) est disposée, vue le long de la sangle principale (3), entre la première zone d'extrémité (7) et la deuxième zone d'extrémité (8), la longueur, la largeur et la hauteur de la partie de contact (4) étant dimensionnées de telle sorte que dans un état d'utilisation du dispositif, la partie de contact (4) puisse être disposée sur le patient (1) de façon que la partie de contact (4) passe sur le dos du patient (1), puis sous les aisselles vers le haut et enfin sur l'arrière derrière la tête du patient (1), en se croisant en un point de croisement (9), ou dans le dos derrière la tête du patient (1) en se rapprochant en un point de rapprochement, **caractérisé en ce qu'**un élément de fixation (13) est prévu pour fixer le point de croisement (9) ou le point de rapprochement dans l'état d'utilisation.

2. Dispositif selon la revendication 1, **caractérisé en ce que** la longueur, la largeur et la hauteur de la partie de contact (4) sont dimensionnées de telle façon que dans l'état d'utilisation du dispositif, la partie de contact (4) puisse être disposée sur le patient (1) de telle façon que la partie de contact (4) passe sur le dos du patient (1) en dessous des omoplates de celui-ci.

3. Dispositif selon l'une des revendications 1 à 2, **caractérisé en ce que** l'élément de fixation (13) est refermable.

4. Dispositif selon l'une des revendications 1 à 3, **caractérisé en ce que** l'élément de fixation (13) est conçu comme une bande flexible.

5. Dispositif selon la revendication 4, **caractérisé en ce que** l'élément de fixation (13) présente une fermeture par velours et crochet (15) pour le refermer.

6. Dispositif selon l'une des revendications 4 à 5, **caractérisé en ce que** l'élément de fixation (13) présente au moins un anneau en forme de D (14), de préférence en métal.

7. Dispositif selon l'une des revendications 4 à 6, **caractérisé en ce que** la bande de l'élément de fixation (13) est faite de microfibres.

8. Dispositif selon l'une des revendications 1 à 7, **caractérisé en ce que** les parties d'attache (5, 6) présentent chacune une fermeture par velours et crochet (15, 17) afin de pouvoir attacher la sangle principale (3) sur les éléments de fixation (18, 18').

9. Dispositif selon l'une des revendications 1 à 8, **caractérisé en ce que** la sangle principale (3) est faite au moins en partie de microfibres.

10. Dispositif selon la revendication 9, **caractérisé en ce que** les parties d'attache (5, 6) de la sangle principale (3) sont réalisées chacune comme une bande faite de microfibres.

11. Dispositif selon l'une des revendications 1 à 10, **caractérisé en ce que** la première partie d'attache (5) et/ou la deuxième partie d'attache (6) ont chacune une longueur qui représente entre 1/10^{ème} et 1/5^{ème} de la longueur totale de la sangle principale (3).

12. Système comprenant un dispositif selon l'une des revendications 1 à 11 et un support formé d'un lit (2) avec un matelas (10) et un sommier (11) sur lequel le matelas (10) repose dans l'état d'utilisation du dispositif, la sangle principale (3) étant attachée avec les parties d'attache (5, 6) à des éléments de fixation (18, 18') fixés au sommier (11) dans l'état d'utilisation du dispositif.

13. Système selon la revendication 12, **caractérisé en ce que** les éléments de fixation (18, 18') présentent chacun une fermeture par velours et crochet (15, 16) pour attacher la partie d'attache (5, 6) correspondante de la sangle principale (3).

14. Système selon l'une des revendications 12 à 13, **caractérisé en ce que** les éléments de fixation (18, 18') sont conformés chacun comme une bande et présentent chacun une fermeture par velours et crochet (15, 16, 17) pour la fixation de chaque élément de fixation (18, 18') au support (2).

15. Système selon l'une des revendications 12 à 14, **caractérisé en ce que** les éléments de fixation (18, 18') sont conformés chacun comme une bande faite de microfibres.
